# EUROPEAN PATENT SPECIFICATION

(11) **EP 3 738 566 B1**
(45) Date of publication and mention of the grant of the patent: **21.02.2024**
(21) Application number: 19761566.9
(22) Date of filing: 09.01.2019
(51) Int. Cl.: A61F 13/56, A61F 13/49, A61F 13/513, A61F 13/62

(54) **LOW-WEIGHT INFANT DIAPER**
WINDEL FÜR KLEINKINDER MIT GERINGEM KÖRPERGEWICHT
COUCHE POUR NOURRISSONS DE FAIBLE POIDS

(30) Priority: 28.02.2018 JP 2018035687
(43) Date of publication of application: 18.11.2020
(73) Proprietor: Unicharm Corporation, Ehime 799-0111 (JP)
(72) Inventor: TSUJII, Maki, Kanonji-shi, Kagawa 769-1602 (JP); YAMANAKA, Yasuhiro, Kanonji-shi, Kagawa 769-1602 (JP); SAKAGUCHI, Satoru, Kanonji-shi, Kagawa 769-1602 (JP)
(74) Representative: Dolleymores
(86) International application number: PCT/JP2019/000393
(87) International publication number: WO 2019/167435

(56) References cited:
- WO-A1-2015/046632
- WO-A1-2016/121236
- JP-A- H06 218 009
- JP-A- H06 218 010
- JP-A- 2003 290 286
- JP-A- 2004 089 614
- JP-A- 2005 304 605
- JP-A- 2013 172 814
- JP-U- 3 059 442

## Description

### [Technical Field]

The present invention relates to a low-weight infant diaper.

### [Background Art]

Patent Document 1 discloses an adult incontinence product that includes a clothing shell (hereinafter "main body portion"), which includes an absorbent core, and a pair of strap members (hereinafter "belt members"). Fasteners (e.g., hook members) are provided on the front end portions and the back end portions of the belt members, and attachment pads (e.g., loop members) are provided on the front waist portion and the back waist portion of the main body portion. Accordingly, the front end portions of the belt members can be detachably engaged with the front waist portion of the main body portion, and the back end portions of the belt members can be detachably engaged with the back waist portion of the main body portion.

Low-weight infants, who are born with a low body weight, sometimes sleep in a prone posture. In the case of low-weight infants, there is a concept of "minimal handling" in which it is desirable to have as little contact with a low-weight infant's body as possible. In view of this, it is desirable for a diaper for low-weight infants to employ the belt members of Patent Document 1. In this case, the belt members can be easily detached regardless of the posture of the low-weight infant, and the low-weight infant's diaper can be easily replaced with as little contact with their body as possible.

### [Citation List]

### [Patent Literature]

[Patent Document 1] Japanese Patent Application Publication No. H6-218009
JP 2005304605 A discloses a disposable diaper that can be detached from the stomach side or the back side of a wearer. A mounting tape is provided with fixing portions on both side portions in the longitudinal direction of the tape material. A front target tape is provided in a front waist portion.
JP 2013172814 A also relates to a tape-type disposable diaper. The fastening tapes have a root portion fixed to a back waist portion.
JP 2004089614 A relates to a disposable diaper provided with a variable pattern that changes when it comes into contact with moisture.

### [Summary of Invention]

### [Technical Problem]

However, if attachment pads are provided on the main body portion in correspondence with the fasteners (hereinafter "locking members") of the belt members as in Patent Document 1, the attachment pads commensurately raise the stiffness of the main body portion and may irritate the low-weight infant's delicate skin. In view of this, it is desirable that attachment pads are not provided on the main body portion, and that the locking members of the belt members can be directly engaged with the non-skin-side surface (i.e., the exterior sheet) of the main body portion.

However, in this case, it is difficult to know which positions on the exterior sheet are the positions where the belt members are intended to be locked. For this reason, when a nurse or the like puts such a diaper on a low-weight infant by locking the belt members to positions on the exterior sheet that they think are appropriate, problems are likely to occur if the positions where the belt members are locked are not appropriate, such as the belt members being too short, and the belt members pressing against the low-weight infant's legs. If the belt members are detached and re-locked each time such a case occurs, diaper replacement becomes time-consuming.

In view of this, and an aspect of the present invention is to provide a low-weight infant diaper that can suppress irritation of a low-weight infant's skin while also being able to be replaced smoothly.

### [Solution to Problem]

The present invention provides the low-weight infant diaper of independent claim 1. The dependent claims specify preferred but optional features.

Features of the present invention other than the above will become clear by reading the description of the present specification with reference to the accompanying drawings.

### [Advantageous Effects of Invention]

According to the present invention, it is possible to provide a low-weight infant diaper that can suppress irritation of a low-weight infant's skin while also being able to be replaced smoothly.

### [Brief Description of the Drawings]

FIG. 1 is a plan view of a skin side of a low-weight infant diaper 1 in an unfolded and stretched state.
FIG. 2A is a cross-sectional view along a line A-A shown in in FIG. 1, and FIG. 2B is a cross-sectional view along a line B-B shown in FIG. 1.
FIG. 3 is an illustrative diagram of a posture unique to low-weight infants.
FIG. 4 is an illustrative diagram of non-skin-side surfaces of a pair of belt members 20 and a main body portion 10.
FIG. 5A is an illustrative diagram of a marker 30 of the belt member 20, and FIG. 5B is a diagram showing a state where the belt member 20 has been locked to the main body portion 10 based on the marker 30.
FIG. 6A to 6C are diagrams showing a state where the belt member 20 has been locked to the main body portion 10 based on the marker 30.
FIG. 7 is a diagram showing a state where the belt members 20 have been locked to the main body portion 10 based on the markers 30.
FIGS. 8A to 8C are illustrative diagrams of forms of the diaper 1 during packaging.
FIGS. 9A to 9C are illustrative diagrams of markers 35 to 37 according to variations.
FIG. 10 is a plan view of a low-weight infant diaper 50 according to a variation in an unfolded and stretched state as viewed from a skin side.

### [Description of Embodiments]

At least the following matters will become clear with the description of this specification and the attached drawings.

A low-weight infant diaper including:
an absorbent body;
an exterior sheet arranged on a non-skin side with respect to the absorbent body;
a pair of belt members,
   the pair of belt members each including:
      a locking member on a one side in a longitudinal direction of the belt member and
      a locking member on an other side in the longitudinal direction of the belt member,
   the pair of belt members being capable of being locked to a non-skin-side surface of the exterior sheet with use of the locking members, the locking members being directly lockable to the exterior sheet; and
a marker that indicates a lock position for one or more of the belt members on the non-skin-side surface of the exterior sheet,
   the marker being visible from a non-skin-side surface side of the exterior sheet.

According to this low-weight infant diaper, the locking members are directly locked to the exterior sheet, thus preventing an increase in the stiffness of the exterior sheet and making it possible to suppress irritation of the low-weight infant's skin. Also, the belt members can be locked at appropriate positions on the exterior sheet based on the marker, and therefore the diaper can be replaced smoothly.

In the low-weight infant diaper of the present invention,
a plurality of the markers includes
a first marker arranged on a first side in a longitudinal direction of the exterior sheet and
a second marker arranged on a second side in the longitudinal direction of the exterior sheet.

According to the low-weight infant diaper of the present invention, the belt members can be locked at appropriate positions on both the first side and the second side in the longitudinal direction of the exterior sheet.

In such a low-weight infant diaper,
the pair of belt members are locked to the non-skin-side surface of the exterior sheet such that the longitudinal direction of the belt members is aligned with a transverse direction of the exterior sheet,
the pair of belt members cover an entirety of the first marker, and
outward portions of the belt members in the transverse direction of the exterior sheet are folded inward, and are located between portions of the absorbent body in a state where the absorbent body has been folded in the longitudinal direction of the exterior sheet.

According to this low-weight infant diaper, the first marker is not exposed, thus suppressing the case where the marker impairs the overall design of the diaper. Because the second marker is exposed, the user (nurse or the like) can easily recognize the marker.

In such a low-weight infant diaper,
in a state where the exterior sheet has been folded in the longitudinal direction of the exterior sheet,
   two ends of the exterior sheet are not aligned with each other, and
the first marker and the second marker have an overlapping portion overlapped in a direction in which portions of the folded exterior sheet are overlaid, as the exterior sheet is viewed from the non-skin side.

According to this low-weight infant diaper, the amount of misalignment between the first marker and the second marker in the longitudinal direction of the exterior sheet is small, thus allowing the user to easily recognize that the two markers both indicate lock positions for the belt members.

In such a low-weight infant diaper,
a plurality of the markers are symmetric in terms of shape and arrangement position with respect to a center line that bisects the exterior sheet in a transverse direction of the exterior sheet.

According to this low-weight infant diaper, the pair of belt members can be locked in a balanced manner.

In such a low-weight infant diaper,
a plurality of the markers includes
   a one-side marker indicating a lock position for one belt member out of the pair of belt members and
   an other-side marker indicating a lock position for an other belt member out of the pair of belt members, and
the one-side marker and the other-side marker are arranged with a space in the transverse direction of the exterior sheet.

According to this low-weight infant diaper, it is easy to recognize the lock positions for the belt members in the transverse direction of the exterior sheet.

In such a low-weight infant diaper,
the pair of belt members are locked to the non-skin-side surface of the exterior sheet such that the longitudinal direction of the belt members is aligned with a transverse direction of the exterior sheet, and
the size of a portion of the one-side marker that is exposed from the one belt member is different from the size of a portion of the other-side marker that is exposed from the other belt member.

According to this low-weight infant diaper, the user can easily recognize that the belt members can be attached and detached in alignment with the markers.

In such a low-weight infant diaper,
concerning an inward end portion that is of each of the belt members and that is located inward in the transverse direction of the exterior sheet,
   the low-weight infant diaper has a position where the inward end portion is to be located when the belt member has been locked to the exterior sheet,
concerning an inward end portion that is of the one-side marker and that is located inward in the transverse direction of the exterior sheet,
   the inward end portion of the one-side marker is arranged at the position where the inward end portion of the one belt member are to be located, and
concerning an inward end portion that is of the other-side marker and that is located inward in the transverse direction of the exterior sheet,
   the inward end portion of the other-side marker is arranged at the position where the inward end portion of the other belt member are to be located.

According to this low-weight infant diaper, the belt members can be easily locked at appropriate positions in the transverse direction of the exterior sheet.

In such a low-weight infant diaper,
the pair of belt members are locked to the non-skin-side surface of the exterior sheet such that the longitudinal direction of the belt members is aligned with a transverse direction of the exterior sheet,
the inward end portion of the one-side marker has an overlap portion that is overlapped in the transverse direction of the exterior sheet with the inward end portion of the one belt member, and
the inward end portion of the other-side marker has an overlap portion that is overlapped in the transverse direction of the exterior sheet with the inward end portion of the other belt member.

According to this low-weight infant diaper, the diaper can be easily put on in a state where the belt members have been locked at appropriate positions in the transverse direction of the exterior sheet.

In such a low-weight infant diaper,
the one-side marker and the other-side marker are arranged outward in the transverse direction of the exterior sheet with respect to the absorbent body.

According to this low-weight infant diaper, the belt members are less likely to be locked at uneven portions at the side ends of the absorbent body, thus making it possible to suppress detachment of the belt members caused by such unevenness.

In such a low-weight infant diaper,
the marker is shaped as a straight line that is elongated in a transverse direction of the exterior sheet.

According to this low-weight infant diaper, the ends extending along the longitudinal direction of the belt members can be easily aligned with the markers.

In such a low-weight infant diaper,
concerning an outward end that is of each of the belt members and that is located outward in a longitudinal direction of the exterior sheet,
   the low-weight infant diaper has a position where the outward end is to be located when the belt member has been locked to the exterior sheet, and
the marker is arranged at the position where the outward end of the belt member is to be located.

According to this low-weight infant diaper, the belt members can be easily locked at an appropriate position in the longitudinal direction of the exterior sheet.

In such a low-weight infant diaper,
the pair of belt members are locked to the non-skin-side surface of the exterior sheet such that the longitudinal direction of the belt members is aligned with a transverse direction of the exterior sheet,
the outward end of the belt member is located closer to the marker in the longitudinal direction of the exterior sheet than inward end of the belt member is, and
the marker has a portion that extends along the outward end of the belt member.

According to this low-weight infant diaper, the diaper can be easily put on in a state where the belt members have been locked at an appropriate position in the longitudinal direction of the exterior sheet.

In such a low-weight infant diaper,
a design mark is provided on a central side in the longitudinal direction of the exterior sheet with respect to the marker, and
the design mark is visible from the non-skin-side surface side of the exterior sheet.

According to this low-weight infant diaper, the markers and the design marks are less likely to be confused with each other, and the markers can be recognized easily.

In such a low-weight infant diaper,
the marker has an overlap portion that is overlapped in a longitudinal direction of the exterior sheet with an end portion that is of the absorbent body and that is located in the longitudinal direction of the exterior sheet.

According to this low-weight infant diaper, portions that include the absorbent body are fixed to (in close contact with) the low-weight infant's body by the belt members, and gaps are less likely to be formed between the diaper and the low-weight infant's body, making it possible to the leakage of excrement.

In such a low-weight infant diaper,
the marker has a portion that indicates a plurality of lock positions for one of the belt members.

According to this low-weight infant diaper, the belt members can be locked at appropriate positions in accordance with characteristics of the low-weight infant's body shape.

### Embodiment

A low-weight infant diaper of the present embodiment is a diaper for use by infants whose body weight is 3000 grams or lower, and particularly lower than 2500 grams.

### Basic configuration of low-weight infant diaper 1

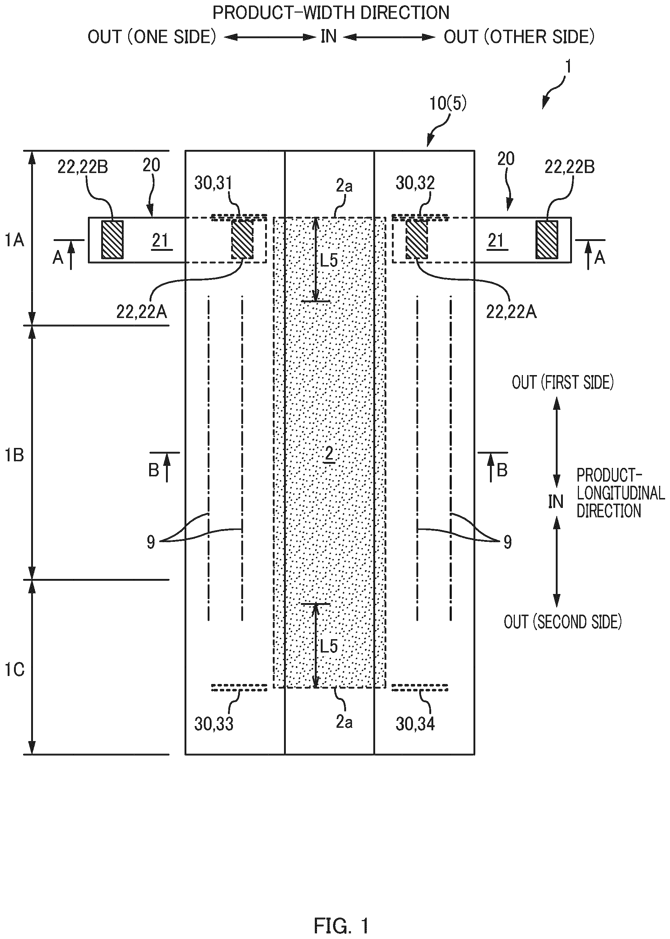

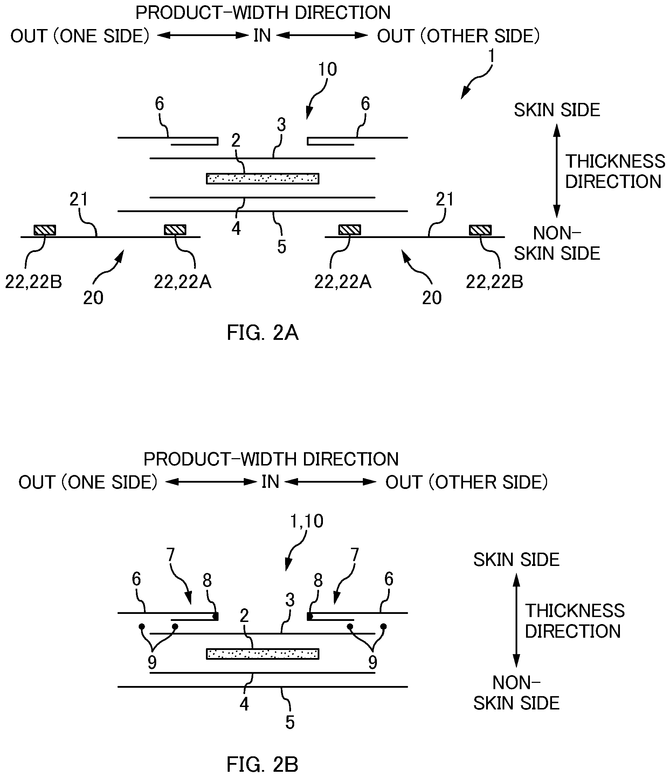

FIG. 1 is a plan view of the skin side of a low-weight infant diaper 1 (hereinafter, also called the "diaper") in an unfolded and stretched state. FIG. 2A is a cross-sectional view along a line A-A shown in in FIG. 1, and FIG. 2B is a cross-sectional view along a line B-B shown in FIG. 1. Note that the stretched state of the diaper 1 is a state in which the diaper 1 has been stretched to the extent that wrinkles are substantially no longer visible, and is a state in which the diaper 1 has been stretched such that the dimensions of constituent members (e.g., a later-described top sheet 3) of the diaper 1 match or are close to the dimensions of the members on their own.

The diaper 1 has a product-longitudinal direction and a product-width direction that are orthogonal to each other, and includes a main body portion 10 and a pair of belt members 20. As shown in FIG. 2A, the direction in which constituent members of the diaper 1 are overlaid on each other will be called the thickness direction. With respect to the thickness direction, the side that comes into contact with the wearer is the skin side, and the opposite side is the non-skin side. The diaper 1 also includes a first waist portion 1A, a crotch portion 1B, and a second waist portion 1C that are arranged side-by-side in the product-longitudinal direction.

As shown in FIGS. 2A and the like, the main body portion 10 includes an absorbent body 2 that absorbs excreted fluid, a liquid-permeable top sheet 3 arranged on the skin side with respect to the absorbent body 2, a liquid-impermeable leak-proof sheet 4 arranged on the non-skin side with respect to the absorbent body 2, an exterior sheet 5 arranged on the non-skin side with respect to the leak-proof sheet 4, and a pair of side sheets 6 provided at the two side portions of the top sheet 3 in the product-width direction.

Examples of the absorbent body 2 include: an absorbent body obtained by shaping liquid-absorbent fibers, such as pulp fibers containing a superabsorbent polymer (so-called SAP), into a predetermined shape; and an SAP sheet obtained by affixing an SAP layer to a hydrophilic sheet. The surface of the absorbent body 2 may be constituted by a core-wrapping sheet made of liquid-permeable tissue paper or nonwoven fabric.

The planar shape of the main body portion 10, that is to say the planar shape of the exterior sheet 5, is rectangular, and the main body portion 10 and the exterior sheet 5 have a longitudinal direction and a transverse direction that are orthogonal to each other. The longitudinal direction of the main body portion 10 and the exterior sheet 5 correspond to the product-longitudinal direction of the diaper 1, and the transverse direction of the main body portion 10 and the exterior sheet 5 correspond to the product-width direction of the diaper 1. In the following description, the longitudinal direction of the exterior sheet 5 will also be called the product-longitudinal direction, and the transverse direction of the exterior sheet 5 will also be called the product-width direction.

A barrier cuff portion 7 is provided in each of two side portions of the diaper 1 in the product-width direction. In each of the barrier cuff portions 7, a portion of the side sheet 6 rises to the skin side due to a barrier-cuff-portion elastic member 8 (see FIG. 2B) that stretches and contracts in the product-longitudinal direction. Also, leg elastic members 9 that stretch and contract in the product-longitudinal direction are provided in each of two side portions of the diaper 1 in the product-width direction, and the diaper 1 thus fits around the wearer's legs.

The belt members 20 each include a rectangular base member 21 and two locking members 22 (22A and 22B) that are attached to the same surface (skin-side surface) of the base member 21. The locking members 22A and 22B are respectively attached to a first-side end portion of the base member 21 on one side in the longitudinal direction of the belt member 20, and a second-side end portion on an other side in the longitudinal direction.

The belt member 20 can be detachably locked to the non-skin-side surface of the exterior sheet 5 due to the locking members 22. As one example, the locking members 22 are hook members of a hook-and-loop fastener, and the exterior sheet 5 is a nonwoven fabric sheet.

Also, it is desirable that the diaper 1 of the present embodiment, which is for low-weight infants, has a product length (dimension when the diaper 1 is stretched in the product-longitudinal direction so as to eliminate wrinkles) of approximately 170 to 330 mm. For example, it is desirable that the product length is 310 mm in the case of a body weight lower than 2500 g, 270 mm in the case of a body weight lower than 1500 g, and 230 mm in the case of a body weight lower than 1000 g.

Also, it is desirable that the waist dimension of the diaper 1 is approximately 160 to 295 mm. The waist dimension is the dimension in the case where the product-width-direction outward end portion of one belt member 20 has been aligned with the product-width-direction inward end portion of the other belt member 20, and the diaper 1 has been stretched in the product-width direction so as to eliminate wrinkles. For example, it is desirable that the waist dimension is 273.5 mm in the case of a body weight lower than 2500 g, and 220 mm in the case of a body weight lower than 1500 g.

### Replacement of low-weight infant diaper 1

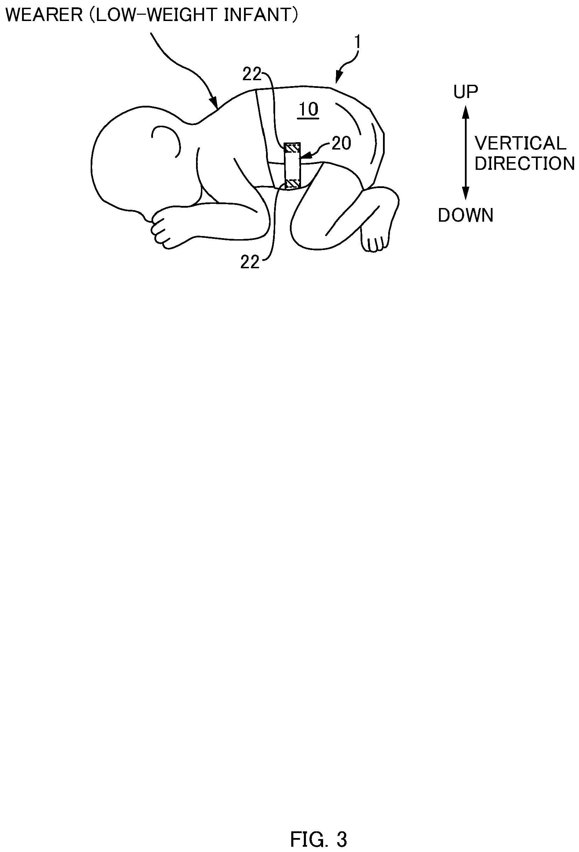

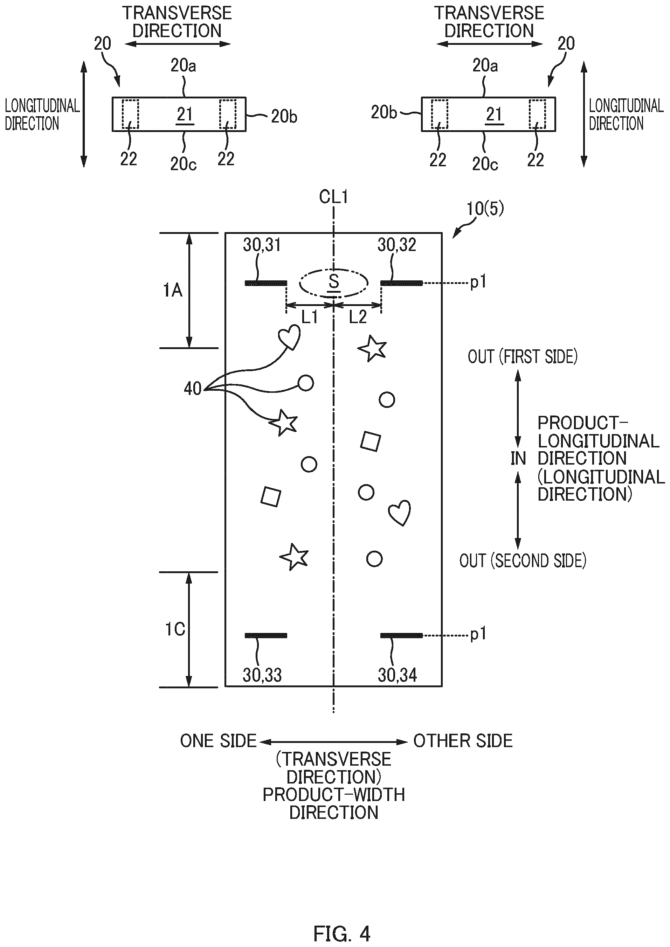

FIG. 3 is an illustrative diagram of a posture unique to low-weight infants. FIG. 4 is an illustrative diagram of non-skin-side surfaces of the pair of belt members 20 and the main body portion 10.

As previously described, the belt members 20 each include the locking members 22 on one (first) side and the other (second) side in the longitudinal direction. For this reason, the user can separate the belt members 20 from the main body portion 10 as shown in FIG. 4, and can attach the belt members 20 to the main body portion 10 as shown in FIG. 1.

When the diaper 1 is to be put on, as shown in FIG. 1, the locking members 22A of the belt members 20 are locked to the non-skin-side surface of the exterior sheet 5 in the first waist portion 1A, for example. Specifically, the longitudinal direction of the belt members 20 is aligned with the product-width direction of the diaper 1, and the transverse direction of the belt members 20 is aligned with the product-longitudinal direction of the diaper 1.

The user then folds the diaper 1, which is in the state shown in FIG. 1, at approximately the center in the product-longitudinal direction arranging it in accordance with the wearer. The portions of the belt members 20 that extend outward from the exterior sheet 5 in the product-width direction are then folded inward in the product-width direction by the user. Lastly, the user locks the locking members 22B of the belt members 20 to the non-skin-side surface of the exterior sheet 5 in the second waist portion 1C for example, thus fixing the position of the diaper 1 relative to the low-weight infant.

Note that because low-weight infants have a small waist, there are cases where the portions of the pair of belt members 20 that extend from the exterior sheet 5 are locked in an overlapping state. In other words, there are cases where one of the two belt members 20 is locked to the non-skin-side surface of the exterior sheet 5, and the other belt member 20 is locked to the non-skin-side surface of the one belt member 20. For this reason, it is preferable that the locking members 22 can also be locked to the non-skin-side surfaces of the belt members 20 (base members 21). For example, the non-skin-side surfaces of the base members 21 may be constituted by nonwoven fabric.

Also, as shown in FIG. 3, low-weight infants often maintain a prone posture in which their back curves in a C shape and their legs are sharply bent in an M shape. In the case of low-weight infants, there is a concept of "minimal handling" in which it is desirable to have as little contact as possible with a low-weight infant's body. For this reason, when the diaper 1 is replaced, there are cases where the low-weight infant is kept in the prone posture instead of being turned faced up when replacing the diaper 1.

With an ordinary tape-type diaper, the belt members are non-detachably fixed to a part of the waist portion that is in contact with the wearer's back side. For this reason, when an ordinary tape-type diaper is used with a low-weight infant in the prone posture, it is necessary to place one hand under the low-weight infant's stomach in order to lock or detach the belt members. In this case, replacement of the diaper 1 is not easy. In particular, in the case where a low-weight infant has been placed in an incubator, a nurse or the like needs to insert their arms through the window of the incubator and perform the task in a small space, thus making replacement of the diaper 1 even more difficult.

In contrast, with the diaper 1 of the present embodiment, both the first-side end portions and the second-side end portions of the belt members 20 in the longitudinal direction can be detached from the main body portion 10. For this reason, the locking and detachment of the belt members 20 can be performed on the back side of the low-weight infant in the prone posture. More specifically, in a state of the diaper 1 in which the belt members 20 are locked to the non-skin-side surface of the part of the waist portion that comes into contact with the low-weight infant's stomach region, the diaper 1 is passed between the low-weight infant's leg when in the prone posture, and then the diaper 1 is folded, thereafter making it possible to lock the belt members 20 on the back side of the low-weight infant.

Accordingly, the burden placed on the nurse or the like can be mitigated, and the diaper 1 can be replaced smoothly. Also, regardless of which posture the low-weight infant is in, the diaper 1 can be replaced with as little change to the low-weight infant's posture as possible, and therefore the burden placed on the low-weight infant can also be mitigated.

### Marker 30 of belt member 20

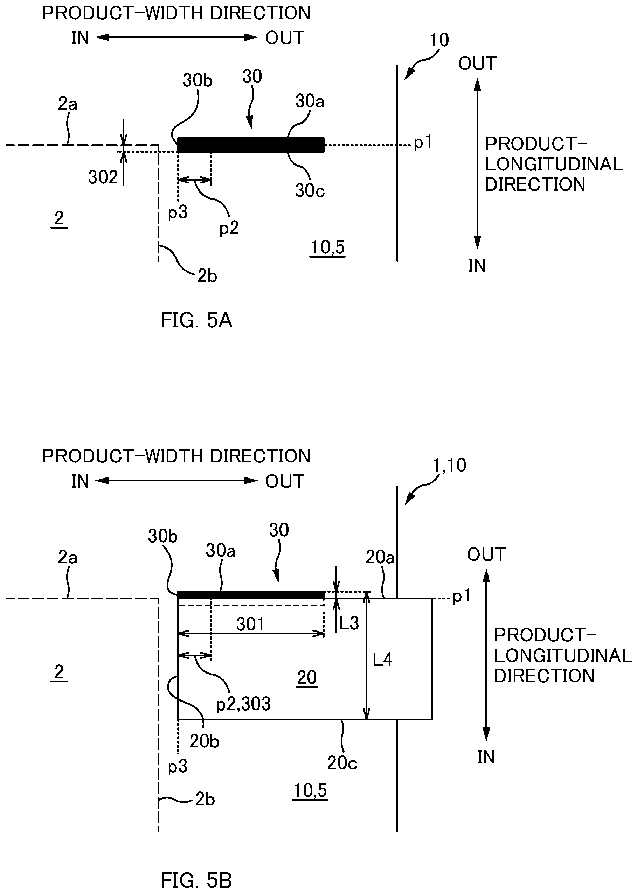

FIG. 5A is an illustrative diagram of a marker 30 of the belt member 20. FIGS. 5B, 6A to 6C, and 7 are diagrams showing a state where the belt member 20 has been locked to the main body portion 10 based on the marker 30.

Members for locking of the locking members 22 of the belt members 20 (e.g., hook-and-loop fastener type loop members) are not provided separately from the exterior sheet 5 in the diaper 1 of the present embodiment, and the locking members 22 of the belt members 20 are directly locked to the non-skin-side surface of the exterior sheet 5. For this reason, the stiffness of the main body portion 10 does not increase due to the provision of such loop members, and it is possible to suppress irritation of the low-weight infant's skin.

However, in the case where loop members are not provided, it is difficult to know the positions where the belt members 20 are to be locked on the exterior sheet 5. For example, as shown in FIG. 4, if the diaper 1 is sold in the state where the belt members 20 are not attached to the main body portion 10, the nurse or the like who is to replace the diaper 1 will lock the belt members 20 to positions that they think are appropriate on the exterior sheet 5.

Also, when the diaper 1 is put on, the unfolded diaper 1 is placed underneath the low-weight infant who is in the supine or prone posture, and at this time, the side of the diaper 1 where the belt members 20 are locked (the first waist portion 1A side in FIG. 1) is normally arranged at the front. For this reason, the belt members 20 sometimes become caught on the sheets or the like and become detached from the main body portion 10. In such cases as well, the nurse or the like will lock the belt members 20 to positions that they think are appropriate on the exterior sheet 5.

However, if the belt members 20 are locked at unsuitable positions, problems occur such as the belt members 20 being too short, and the belt members 20 pressing against the low-weight infant's legs. In such a case, the nurse or the like pulls the diaper 1 out from under the low-weight infant, re-locks the belt members 20 to the exterior sheet 5, and then places diaper 1 underneath the low-weight infant again. Repeating such operations makes the replacement of the diaper 1 time-consuming.

In view of this, the diaper 1 of the present embodiment is provided with markers 30 that indicate locking positions for the belt members 20 on the non-skin-side surface of the exterior sheet 5. The markers 30 are visible from the non-skin-side surface side of the exterior sheet 5 (i.e., from the outside of the diaper 1). In the present embodiment, the markers 30 are printed on the non-skin-side surface of the leak-proof sheet 4, and the markers 30 can be seen through the exterior sheet 5.

Accordingly, even if the belt members 20 have been separated from the main body portion 10, the nurse or the like can lock to the belt members 20 at appropriate positions on the exterior sheet 5 based on the markers 30. For this reason, the nurse or the like can smoothly replace the diaper 1 without repeatedly adjusting the positions of the belt members 20 and then locking them, and the burden placed on the nurse or the like can be mitigated. It is also possible to reduce the number of times that the diaper 1 is placed or pulled from under the low-weight infant, and irritation of the low-weight infant's skin can be suppressed.

Also, due to the provision of the markers 30 on the diaper 1, even a family member who has less experience with replacement of a low-weight infant diaper than a nurse can lock the belt members 20 at appropriate positions on the exterior sheet 5 based on the markers 30.

Note that the markers 30 are not limited to be provided on the leak-proof sheet 4, and may be printed on the non-skin-side surface of the absorbent body 2, the exterior sheet 5, or the like. Also, if the diaper 1 has design marks 40 (see FIG. 4), the markers 30 may be printed along with the design marks 40 in the step for printing the design marks 40. Also, the markers 30 are not limited to being formed by printing (dying), and may be formed by an embossed pattern (recessions) that are visible from the non-skin-side surface side of the exterior sheet 5.

The markers 30 of the present embodiment include four markers 31 to 34 arranged in end portions with respect to the product-width direction and the product-longitudinal direction. The markers 30 (31 to 34) are straight and elongated in the product-width direction (thin rectangles). For this reason, as shown in FIG. 5B, lengthwise ends 20a of the belt member 20 (ends 20a extending along the longitudinal direction of the belt member 20) can be aligned with the longitudinal direction of the marker 30. This makes it easier to lock the belt member 20 to the exterior sheet 5 in alignment with the marker 30.

Also, if the markers 30 are straight and elongated in the product-width direction, the markers 30 can also be used as register marks. Register marks are used when detecting the positions of the design marks 40 (see FIG. 4) of the diaper 1 in the manufacturing line of the diaper 1. For example, material conveying is controlled based on the register marks such that a member such as the absorbent body 2 is placed at an appropriate product-longitudinal-direction position on the member on which the design marks 40 have been printed (e.g., the leak-proof sheet 4) . If the markers 30 are used as register marks, the number of marks other than the decorative design marks 40 is lower than in the case where register marks are provided separately from the markers 30 for example, and diaper 1 has a higher freedom of design.

Also, among the four markers 31 to 34, the one-side markers 31 and 33, which are arranged on the one side in the product-width direction, indicate the lock position for one of the two belt members 20, and the other-side markers 32 and 34, which are arranged on the other side in the product-width direction, indicate the lock position for the other one of the two belt members 20. Also, a space S extending in the product-width direction is provided between the one-side marker 31 and the other-side marker 32, and between the one-side marker 33 and the other-side marker 34.

This thus facilitates recognition of the lock positions for the belt members 20 in the product-width direction. Also, due to the two markers in each pair (31 and 32, 33 and 34) being arranged side-by-side in the product-width direction, the nurse or the like who is looking at the markers 30 can intuitively recognize that the markers 30 are indicating lock positions for the pair of belt members 20. Accordingly, the belt members 20 can be easily locked based on the markers 30.

Also, as shown in FIG. 4, it is desirable that the one-side markers 31 and 33 and the other-side markers 32 and 34 are symmetric in terms of shape and arrangement position with respect to a center line CL1 that bisects the exterior sheet 5 in the transverse direction thereof. Note that the above-described configuration desirably pertains to the unfolded and stretched state of the main body portion 10. Also, the symmetric positions are not required to be in perfect symmetry, and the present invention also encompasses cases of deviation within the range of manufacturing error.

In the present embodiment, the one-side markers 31 and 33 and the other-side markers 32 and 34 are all straight, and with respect to the product-width direction, a length L1 from the center line CL1 to the ends of the one-side markers 31 and 33 is equivalent to a length L2 from the center line CL1 to the ends of the other-side markers 32 and 34.

For this reason, based on the markers 30, the pair of belt members 20 can be locked in a balanced manner in the product-width direction. Accordingly, it is possible to prevent problems such as one of the belt members 20 extending too much or too little from the exterior sheet 5, and consequently the diaper 1 can be put on easily. Also, by looking at the two markers in each pair (31 and 32, 33 and 34) arranged symmetrically in the product-width direction, the nurse or the like can more intuitively recognize that the markers 30 indicate lock positions for the pair of belt members 20.

Also, as shown in FIG. 1, the main body portion 10 and the absorbent body 2 of the diaper 1 according to the present embodiment have shapes that are symmetric with respect to the product-longitudinal direction. For this reason, the first waist portion 1A can be arranged on the front side or the back side of the low-weight infant. In other words, the diaper 1 can be put on in either direction with respect to the front and back. In this case, when the diaper 1 is placed under the low-weight infant, the belt members 20 may be locked to the first waist portion 1A side, or the belt members 20 may be locked to the second waist portion 1B side.

In view of this, in the diaper 1 of the present embodiment, the markers 31 and 32 (first markers) are arranged on the first side in the product-longitudinal direction, and the markers 33 and 34 (second markers) are arranged on the second side in the product-longitudinal direction. Accordingly, the nurse or the like can lock the belt members 20 at appropriate positions on both the first side (the first waist portion 1A side) and the second side (the second waist portion 1B side) in the longitudinal direction of the exterior sheet 5.

Also, as shown in FIG. 5B, in the state where the belt member 20 is locked to the exterior sheet 5, the diaper 1 has a position p1 which is a product-longitudinal-direction position where the outward end 20a of the belt member 20 is to be located. In the following description, with respect to the product-longitudinal direction, the outward end 20a of the belt member 20 will also be called the upper end, the inward end 20c of the belt member 20 will also be called the lower end, an outward end 30a of the marker 30 will also be called the upper end, and an inward end 30c of the marker 30 will also be called the lower end.

The position p1 at which the upper end 20a of the belt member 20 is to be located is a position at which certain problems do not occur such as the lock position of the belt member 20 being too high (too close to the waist side) such that the leg opening of the diaper 1 is too large and allows leakage. It is also a position at which other problems do not occur such as the lock position of the belt member 20 being too low (too close to the crotch side) such that the belt member 20 presses against the low-weight infant's legs.

In the case where the marker 30 is straight and elongated in the product-width direction, the belt member 20 is likely to be locked such that the upper end 20a of the belt member 20 is aligned with the marker 30. For this reason, it is preferable that the marker 30 is arranged at the position p1 where the upper end 20a of the belt member 20 is to be located. In the present embodiment, the center of the marker 30 in the product-longitudinal direction is located at the position p1. Accordingly, the belt member 20 is likely to be locked at an appropriate position on the exterior sheet 5 in the product-longitudinal direction based on the marker 30.

Note that it is sufficient that any portion of the marker 30 is located at the position p1, and for example, the upper end 30a or the lower end 30c of the marker 30 may be located at the position p1.

Also, it is preferable that in the case where the diaper 1 is sold in the state where the markers 30 are located at the position p1 and the belt members 20 are locked to the main body portion 10, the diaper 1 satisfies the following conditions immediately after being removed from the packaging.

First, with respect to the product-longitudinal direction, the upper end 20a of the belt member 20 is located closer to the marker 30 than the lower end 20c is. For example, as shown in FIG. 5B, with respect to the product-longitudinal direction, it is desirable that a length L3 from the upper end 30a of the marker 30 to the upper end 20a of the belt member 20 is shorter than a length L4 from the upper end 30a of the marker 30 to the lower end 20c of the belt member 20.

Second, as shown in FIG. 5B, the marker 30 has a portion 301 that extends along the upper end 20a of the belt member 20. In other words, it is desirable that the marker 30 and the upper end 20a of the belt member 20 are approximately parallel with each other. The term "approximately parallel" refers to the case where the angle formed by the marker 30 and the upper end 20a of the belt member 20 is 45 degrees or less, for example.

Accordingly, the diaper 1 is likely to be put on the low-weight infant in the state where the belt members 20 are locked at appropriate positions on the exterior sheet 5 in the product-longitudinal direction.

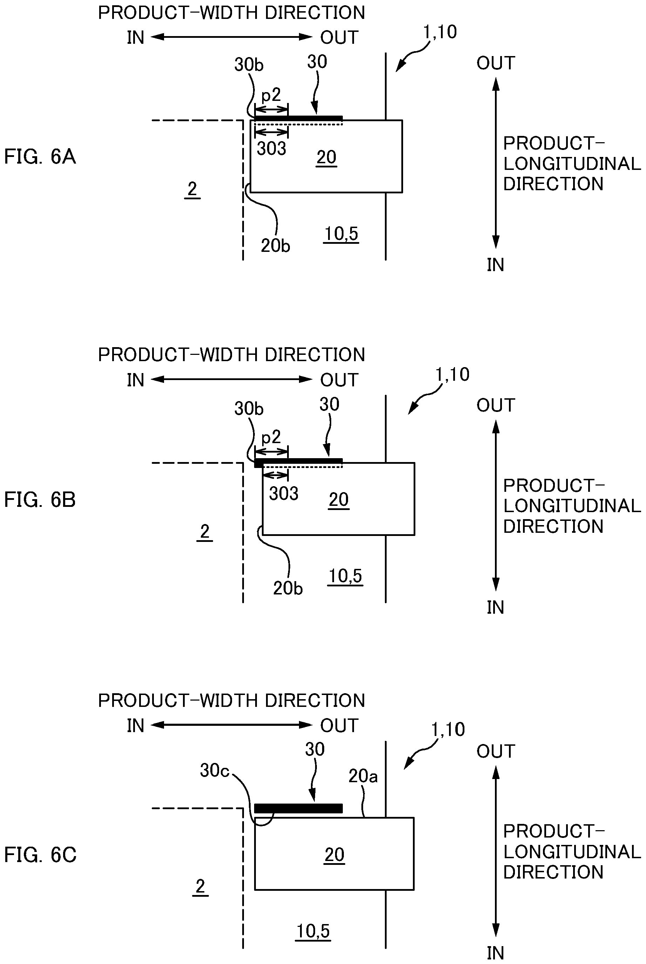

Furthermore, it is preferable that the markers 30 each have a portion that is exposed from the belt member 20 (e.g., the solid black portion in FIG. 5B) . Accordingly, upon looking at the diaper 1 immediately after removal from the packaging, the nurse or the like can recognize that the upper ends 20a of the belt members 20 are aligned with the markers 30. Note that the markers 30 may be partially covered by the belt members 20 as shown in FIG. 5B, or the markers 30 may be completely exposed from the belt members 20 as shown in FIG. 6C.

Note that the present invention is not limited to the above configurations, and the marker 30 may be arranged at the position where the lower end 20c of the belt member 20 is to be located.

Also, in the case where the diaper 1 has the decorative design marks 40 that are visible from the non-skin-side surface side of the exterior sheet 5 (see FIG. 4), it is desirable that the design marks 40 are formed inward (on the central side) in the product-longitudinal direction with respect to the markers 30. In other words, it is desirable that the design marks 40 are provided between the markers 31 and 32 on the first side and the markers 33 and 34 on the second side in the product-longitudinal direction.

Accordingly, the markers 30 and the design marks 40 are not likely to be confused with each other, and the nurse or the like can easily recognize the markers 30. Note that although not shown, it is desirable that text indicating the size, the product name, or the like is also provided inward in the product-longitudinal direction with respect to the markers 30.

Also, as previously described, in the case where the markers 30 are arranged at the position p1 where the upper ends 20a of the belt members 20 are to be located, the markers 30 are arranged at relatively outward positions in the product-longitudinal direction. For this reason, the design marks 40 can be provided over a wider range in the product-longitudinal direction, and the freedom of design of the diaper 1 improves.

Also, it is preferable that the markers 30 each have a portion (e.g., 302 in FIG. 5A) that is overlapped in the product-longitudinal direction with a product-longitudinal-direction end portion of the absorbent body 2. More preferably, the marker 30 is arranged at a position overlapped in the product-longitudinal direction with a product-longitudinal-direction end 2a of the absorbent body 2.

In these cases, the belt members 20 are likely to be locked at positions in the product-longitudinal-direction end portions of the absorbent body 2. For this reason, portions of the main body portion 10 that include the absorbent body 2 are fixed to (in close contact with) the low-weight infant's body by the belt members 20, and gaps are not likely to be formed between the diaper 1 and the low-weight infant's body, making it possible to suppress the leakage of excrement.

As an example of the product-longitudinal-direction end portion of the absorbent body 2, there is a portion extending over the range of a length L5 in the case of the diaper 1 in the unfolded and stretched state shown in FIG. 1; the length L5 being 1/6 of the total length of the absorbent body 2, from the end 2a of the absorbent body 2 in the product-longitudinal direction.

Also, as shown in FIG. 5B, in the state where the belt member 20 is locked to the exterior sheet 5, the diaper 1 has a position p2 and a position p3: the position p2 is a product-width-direction position where the inward end portion of the belt member 20 is to be located, and a position p3 is a product-width-direction position where an inward end 20b of the belt member 20 is to be located.

These positions p2 and p3 are positions where certain problems do not occur such as the lock position of the belt member 20 being too inward in the product-width direction such that the portion of the belt member 20 extending from the exterior sheet 5 is too short. They are also positions at which other problems do not occur such as the lock position of the belt member 20 being too outward in the product-width direction such that the surface area of the portion of the locking member 22 locked to the exterior sheet 5 is too small and the belt member 20 becomes detached from the main body portion 10.

As shown in FIG. 4 and the like, in the case where the one-side markers 31 and 33 are respectively spaced in the product-width direction from the other-side markers 32 and 34, the belt members 20 are in general likely to be locked such that the inward end portions (inward ends 20b) of the belt members 20 are aligned with the inward end portions (inward ends 30b) of the markers 30 in the product-width direction.

For this reason, as shown in FIG. 5A, it is desirable that the inward end portion of the marker 30 in the product-width direction is arranged at the position p2. More preferably, the inward end 30b of the marker 30 in the product-width direction is arranged at the position p3. Accordingly, the belt members 20 are likely to be locked at appropriate positions on the exterior sheet 5 in the product-width direction.

Also, it is preferable that in the case where the diaper 1 is sold in the state where the belt members 20 are locked to the main body portion 10 such that the product-width-direction inward end portions of the markers 30 are arranged at the positions p2, the diaper 1 satisfies the following immediately after removal from the packaging. Specifically, the inward end portion of each of the markers 30 has a portion 303 that is overlapped in the product-width direction with the inward end portion of the corresponding belt member 20.

Accordingly, the diaper 1 is likely to be put on the low-weight infant in the state where the belt members 20 are locked at appropriate positions on the exterior sheet 5 in the product-width direction. Note that the inward end 20b of the belt member 20 may be matched with the inward end 30b of the marker 30 as shown in FIG. 5B, may be located inward in the product-width direction with respect to the inward end 30b of the marker 30 as shown in FIG. 6A, or may be located outward in the product-width direction with respect to the inward end 30b of the marker 30 as shown in FIG. 6B.

Furthermore, it is preferable that the marker 30 has a portion that is exposed from the belt member 20. Accordingly, upon looking at the diaper 1 immediately after removal from the packaging, the nurse or the like can recognize that the inward end portions of the belt members 20 are to be aligned with the inward end portions of the markers 30 in the product-width direction.

As an example of the product-width-direction inward end portion of the marker 30, there is a portion extending over the range of 1/4 the length of the marker 30 from the inward end portion 30b of the marker 30. Also, as an example of the product-width-direction inward end portion of the belt member 20, there is a portion extending over the range from the inward end 20b of the belt member 20 to the position of the inward-side locking member 22A.

Also, in the case where the one-side markers 31 and 33 are respectively spaced in the product-width direction from the other-side markers 32 and 34, it is preferable that the markers 30 are arranged outward with respect to the absorbent body 2 as shown in FIG. 5A.

Accordingly, the belt members 20 are likely to be locked at positions outward in the product-width direction with respect to the absorbent body 2. This makes it possible to prevent the case where the belt members 20 are locked at uneven portions at the side ends 2b of the absorbent body 2 such that the belt members 20 can easily become detached. It is also possible to prevent the belt members 20 from being unnecessarily long.

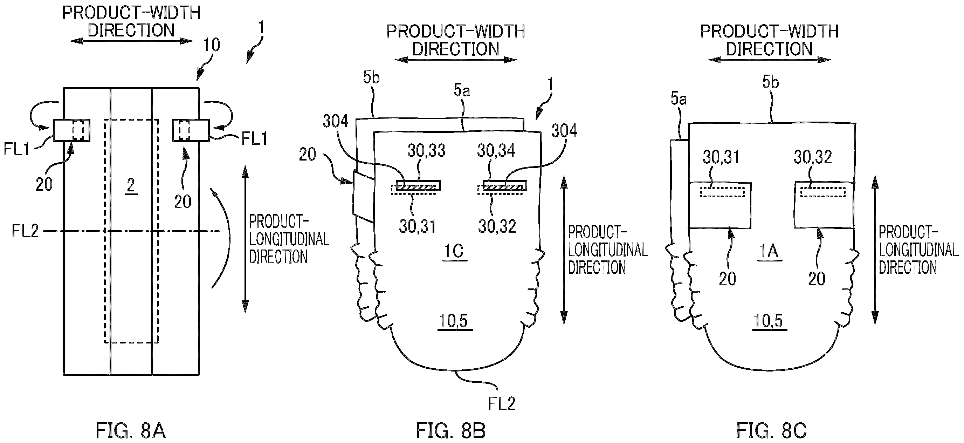

FIGS. 8A to 8C are illustrative diagrams of forms of the diaper 1 during packaging. The diaper 1 may be in any of the following forms during packaging. Specifically, the diaper 1 may be packaged in the state where the pair of belt members 20 are locked to the non-skin-side surface of the exterior sheet 5 such that the longitudinal direction of the belt members 20 is aligned with the transverse direction of the exterior sheet 5, or multiple diapers 1 may be wrapped in the same packaging in the state where the belt members 20 are separate from the main body portion 10, or the diaper 1 may be wrapped in an individual package in the state where the belt members 20 are separate from the main body portion 10.

The following illustrates an example of a method used in the case of packaging in the state where the belt members 20 are locked to the exterior sheet 5. First, as shown in FIG. 8A, outward portions of the belt members 20 in the product-width direction are folded inward at folding positions FL1. Then the main body portion 10 is folded one time at a folding position FL2 at approximately at the center of the main body portion 10 in the product-longitudinal direction. The portions of the belt members 20 that were folded inward in the product-width direction are thus located between portions of the folded main body portion 10 (absorbent body 2), that is to say on the skin-side surfaces of the main body portion 10. The diaper 1 is then packaged in this state (FIGS. 8B and 8C).

In this case, as shown in FIG. 8B, the markers 33 and 34 on the side where the belt members 20 are not locked (e.g., the second waist portion 1C side) are exposed and not covered by the belt members 20. For this reason, as shown in FIG. 8C, it is preferable that the markers 31 and 32 on the side where the belt members 20 are locked (e.g., the first waist portion 1A side) are entirely covered by the pair of belt members 20.

Accordingly, the markers 31 and 32 on one waist portion side are not exposed, thus suppressing the case where these markers 30 impair the overall design of the diaper 1. Also, the markers 33 and 34 on the other waist portion side are exposed, and therefore these markers 30 can be easily recognized by the nurse or the like.

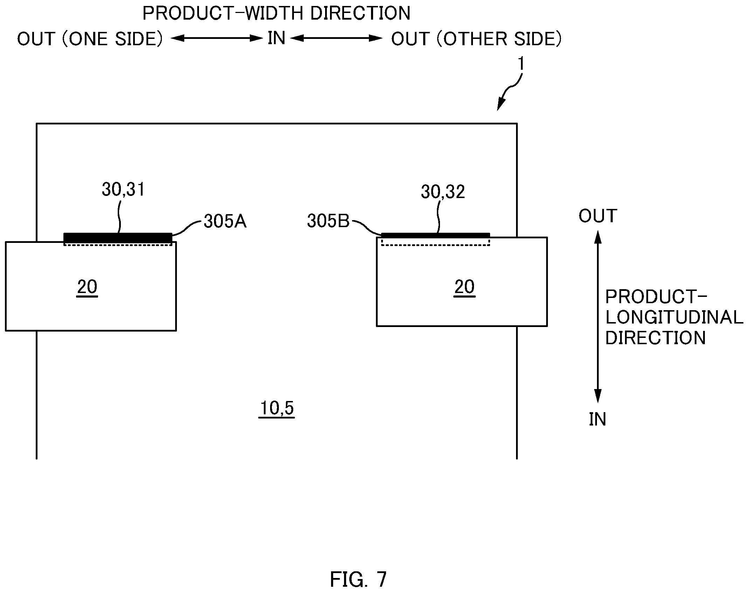

Note that the present invention is not limited to the above configuration, and the markers 31 and 32 on the side where the belt members 20 are locked may be exposed. In this case, as shown in FIG. 7, it is desirable that the size of a portion 305A (portions shown in solid black) of the one-side marker 31 that is exposed from one of the two belt members 20 is different from the size of a portion 305B of the other-side marker 32 that is exposed from the other belt member 20. Accordingly, the nurse or the like can easily recognize that the belt members 20 can be removably attached in alignment with the markers 30.

Also, as shown in FIG. 8B, the following configuration is desirable: the ends 5a and 5b of the folded exterior sheet 5 are not aligned with each other in the product-longitudinal direction; and as the exterior sheet 5 is viewed from the non-skin side, the marker 31 (32) on the one waist portion side and the marker 33 (34) on the other waist portion side have an overlap portion 304 overlapped in the direction in which the folded exterior sheet 5 is overlaid on itself (the thickness direction of the diaper 1).

Specifically, it is desirable that the amount of misalignment in the product-longitudinal direction between the markers 31 and 32 on the one waist portion side and the markers 33 and 34 on the other waist portion side is small. Accordingly, the nurse or the like can easily recognize that the two markers (31 and 32, 33 and 34) are both lock positions for the belt members 20.

There are cases where, when putting the diaper 1 on a low-weight infant, the diaper 1 is folded at the for-packaging folding position FL2. In such a case as well, according to the above configuration, the distance from the folding position FL2 to the marker 31 (32) on the first waist portion side approximately matches the distance from the folding position FL2 to the marker 33 (34) on the second waist portion side. Accordingly, regardless of which markers the belt members 20 are aligned with when locked, the positions of the belt members 20 relative to the low-weight infant are appropriate.

### Variations of marker

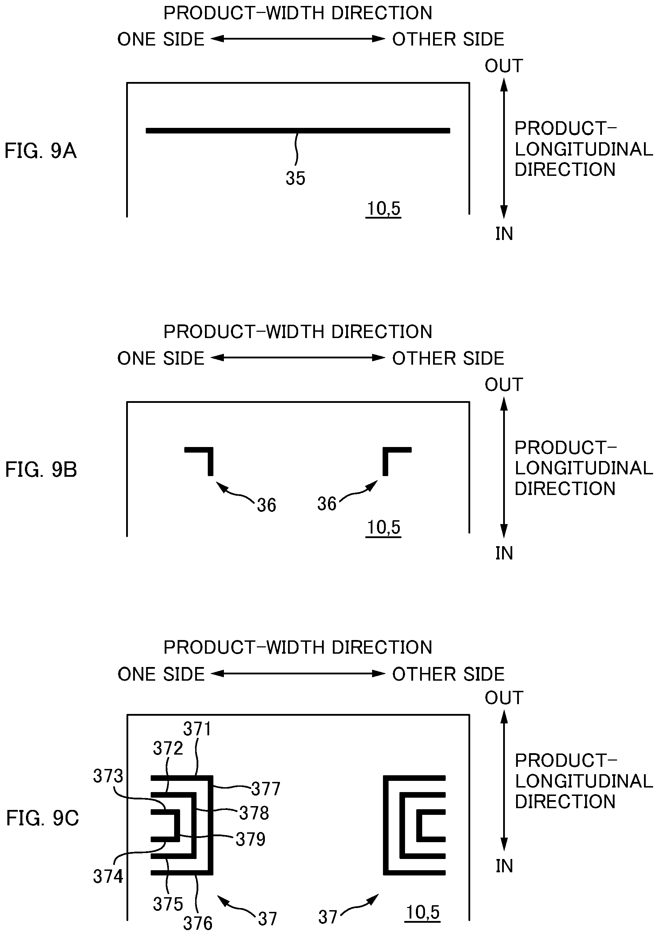

FIGS. 9A to 9C are illustrative diagrams of markers 35 to 37 according to variations.

For example, as shown in FIG. 9A, a marker 35 may be shaped as a straight line that extends continuous from one side of the exterior sheet 5 to the other side. In this case, each pair of belt members 20 is aligned with one marker 35 when being locked.

As another example, as shown in FIG. 9B, markers 36 may be shaped as a right-angled corners. In this case, the corners of the belt members 20 can be aligned with the markers 36.

Also, the markers may have portions that indicate multiple lock positions for a single belt member 20. Accordingly, the nurse or the like can lock the belt members 20 at appropriate positions in accordance with characteristics of the low-weight infant's body shape.

For example, in the case of a marker 37 shown in FIG. 9C, straight lines 371 to 376 that extend in the product-width direction are arranged with gaps therebetween in the product-longitudinal direction, and straight lines 377 to 379 that extend in the product-longitudinal direction are arranged with gaps therebetween in the product-width direction. Accordingly, if the low-weight infant has thin legs, the belt member 20 can be aligned with a portion of the marker on the inward side in the product-longitudinal direction (e.g., 375 or 376), whereas if the low-weight infant has wide legs, the belt member 20 can be aligned with a portion of the marker on the outward side in the product-longitudinal direction (e.g., 371 or 372). Also, if the low-weight infant has a thin waist, the belt member 20 can be aligned with a portion of the marker on the inward side in the product-width direction (e.g., 377), whereas if the low-weight infant has a wide waist, the belt member 20 can be aligned with a portion of the marker on the outward side in the product-width direction (e.g., 379).

### Variation of low-weight infant diaper

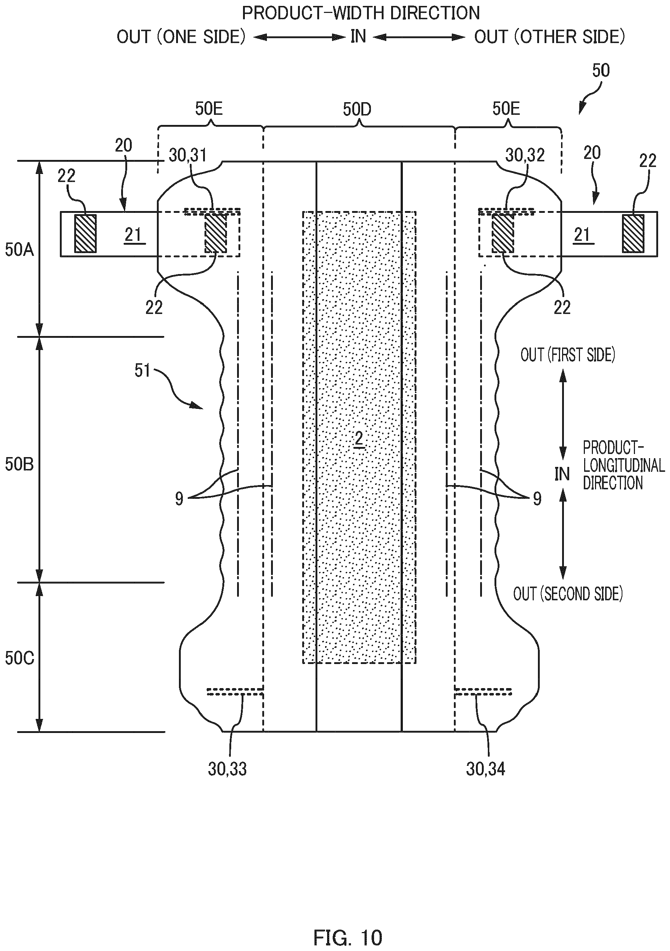

FIG. 10 is a plan view of a low-weight infant diaper 50 according to a variation in an unfolded and stretched state as viewed from the skin side. Although the main body portion 10 (exterior sheet 5) of the low-weight infant diaper 1 is rectangular in the embodiment described above (FIG. 1), there is no limitation to this. For example, as shown in FIG. 10, a main body portion 51 of the low-weight infant diaper 50 may have a central band-shaped region 50D located at the center in the product-width direction, and side flaps 50E located on the sides thereof. The side flaps 50E are formed extending over a first waist portion 50A, a crotch portion 50B, and a second waist portion 50C. The length of the side flaps 50E in the product-width direction in the crotch portion 50B is smaller than the length of the side flaps 50E in the product-width direction in the first waist portion 50A and the second waist portion 50C.

### Other embodiments

Although the embodiment of the present disclosure has been described hereinabove, the above embodiment of the present disclosure is simply to facilitate understanding of the present disclosure and is not in any way to be construed as limiting the present disclosure. The present disclosure may variously be changed or altered without departing from the scope of the invention as defined by the claims.

### [Reference Signs List]

1, 50 diaper (low-weight infant diaper)
2 absorbent body, 3 top sheet, 4 leak-proof sheet,
5 exterior sheet, 6 side sheet,
7 barrier cuff portion, 8 barrier-cuff-portion elastic member,
9 leg elastic member, 10 main body portion,
20 belt member, 21 base member, 22 locking member,
30, 35-37 marker,
31 marker (first marker, first-side marker),
32 marker (first marker, second-side marker),
33 marker (second marker, first-side marker),
34 marker (second marker, second-side marker),
40 design mark

## Claims

1. A low-weight infant diaper (1) comprising:
an absorbent body (2);
an exterior sheet (5) arranged on a non-skin side with respect to the absorbent body (2);
a pair of belt members (20),
the pair of belt members (20) each including:
a locking member (22) on a one side in a longitudinal direction of the belt member and
a locking member (22) on an other side in the longitudinal direction of the belt member,
the pair of belt members (20) being capable of being locked to a non-skin-side surface of the exterior sheet (5) with use of the locking members (22), the locking members being directly lockable to the exterior sheet; and
a marker (30) that indicates a lock position for one or more of the belt members (20) on the non-skin-side surface of the exterior sheet (5),
the marker (30) being visible from a non-skin-side surface side of the exterior sheet (5), wherein
a plurality of the markers (30) includes
a first marker (31, 32) arranged on a first side in a longitudinal direction of the exterior sheet (5) and
a second marker (33, 34) arranged on a second side in the longitudinal direction of the exterior sheet (5).

2. The low-weight infant diaper according to claim 1, wherein
the pair of belt members (20) are locked to the non-skin-side surface of the exterior sheet (5) such that the longitudinal direction of the belt members (20) is aligned with a transverse direction of the exterior sheet (5),
the pair of belt members (20) cover an entirety of the first marker (31, 32), and
outward portions of the belt members (20) in the transverse direction of the exterior sheet (5) are folded inward, and are located between portions of the absorbent body (2) in a state where the absorbent body has been folded in the longitudinal direction of the exterior sheet (5).

3. The low-weight infant diaper according to claim 1 or 2, wherein
in a state where the exterior sheet (5) has been folded in the longitudinal direction of the exterior sheet (5),
two ends of the exterior sheet (5) are not aligned with each other, and
the first marker (31, 32) and the second marker (33, 34) have an overlapping portion overlapped in a direction in which portions of the folded exterior sheet (5) are overlaid, as the exterior sheet (5) is viewed from the non-skin side.

4. The low-weight infant diaper according to any one of claims 1 to 3, wherein
a plurality of the markers (30) are symmetric in terms of shape and arrangement position with respect to a center line (CL1) that bisects the exterior sheet (5) in a transverse direction of the exterior sheet (5).

5. The low-weight infant diaper according to claim 4, wherein
a plurality of the markers (30) includes
a one-side marker (31) indicating a lock position for one belt member out of the pair of belt members (20) and
an other-side marker (32) indicating a lock position for an other belt member out of the pair of belt members (20), and
the one-side marker and the other-side marker are arranged with a space in the transverse direction of the exterior sheet (5).

6. The low-weight infant diaper according to claim 5, wherein
the pair of belt members (20) are locked to the non-skin-side surface of the exterior sheet (5) such that the longitudinal direction of the belt members (20) is aligned with a transverse direction of the exterior sheet (5), and
the size of a portion (305A) of the one-side marker (31) that is exposed from the one belt member is different from the size of a portion (305B) of the other-side marker (32) that is exposed from the other belt member.

7. The low-weight infant diaper according to claim 5 or 6, wherein
concerning an inward end portion (20b) that is of each of the belt members (20) and that is located inward in the transverse direction of the exterior sheet (5),
the low-weight infant diaper has a position (p2) where the inward end portion (20b) is to be located when the belt member has been locked to the exterior sheet (5),
concerning an inward end portion (30b) that is of the one-side marker (31) and that is located inward in the transverse direction of the exterior sheet (5),
the inward end portion (30b) of the one-side marker is arranged at the position where the inward end portion (20b) of the one belt member are to be located, and
concerning an inward end portion (30b) that is of the other-side marker (32) and that is located inward in the transverse direction of the exterior sheet (5),
the inward end portion (30b) of the other-side marker is arranged at the position where the inward end portion (20b) of the other belt member are to be located.

8. The low-weight infant diaper according to claim 7, wherein
the pair of belt members (20) are locked to the non-skin-side surface of the exterior sheet (5) such that the longitudinal direction of the belt members (20) is aligned with a transverse direction of the exterior sheet (5),
the inward end portion (30b) of the one-side marker (31) has an overlap portion that is overlapped in the transverse direction of the exterior sheet (5) with the inward end portion (20b) of the one belt member, and
the inward end portion (30b) of the other-side marker (32) has an overlap portion that is overlapped in the transverse direction of the exterior sheet (5) with the inward end portion (20b) of the other belt member.

9. The low-weight infant diaper according to any one of claims 5 to 8, wherein
the one-side marker (31) and the other-side marker (32) are arranged outward in the transverse direction of the exterior sheet (5) with respect to the absorbent body.

10. The low-weight infant diaper according to any one of claims 1 to 9, wherein
the marker (30) is shaped as a straight line that is elongated in a transverse direction of the exterior sheet (5).

11. The low-weight infant diaper according to claim 10, wherein
concerning an outward end (20a) that is of each of the belt members (20) and that is located outward in a longitudinal direction of the exterior sheet (5),
the low-weight infant diaper has a position (p1) where the outward end is to be located when the belt member has been locked to the exterior sheet (5), and
the marker (30) is arranged at the position where the outward end (20a) of the belt member is to be located.

12. The low-weight infant diaper according to claim 11, wherein
the pair of belt members (20) are locked to the non-skin-side surface of the exterior sheet (5) such that the longitudinal direction of the belt members (20) is aligned with a transverse direction of the exterior sheet (5),
the outward end (20a) of the belt member is located closer to the marker (30) in the longitudinal direction of the exterior sheet (5) than inward end (20b) of the belt member is, and
the marker (30) has a portion that extends along the outward end (20a) of the belt member.

13. The low-weight infant diaper according to claim 11 or 12, wherein
a design mark (40) is provided on a central side in the longitudinal direction of the exterior sheet (5) with respect to the marker (30), and
the design mark (40) is visible from the non-skin-side surface side of the exterior sheet (5).

14. The low-weight infant diaper according to any one of claims 1 to 13, wherein
the marker (30) has an overlap portion that is overlapped in a longitudinal direction of the exterior sheet (5) with an end portion that is of the absorbent body (2) and that is located in the longitudinal direction of the exterior sheet (5).

15. The low-weight infant diaper according to any one of claims 1 to 14, wherein
the marker (30) has a portion that indicates a plurality of lock positions for one of the belt members (20).

## Patentansprüche

1. Leichtgewichtige Säuglingswindel (1), die Folgendes umfasst:
einen Saugkörper (2);
eine äußere Lage (5), die auf einer Nicht-Hautseite in Bezug auf den Saugkörper (2) angeordnet ist;
ein Paar von Gürtelelementen (20),
wobei das Paar von Gürtelelementen (20) jeweils Folgendes einschließt:
ein Verschlusselement (22) auf einer Seite in einer Längsrichtung des Gürtelelements und
ein Verschlusselement (22) auf einer anderen Seite in der Längsrichtung des Gürtelelements,
wobei das Paar von Gürtelelementen (20) unter Verwendung der Verschlusselemente (22) mit einer Nicht-Hautseitenoberfläche der äußeren Lage (5) verschlossen werden kann, wobei die Verschlusselemente direkt mit der äußeren Lage verschließbar sind; und
einen Marker (30), der eine Verschlussposition für ein oder mehrere der Gürtelelemente (20) auf der Nicht-Hautseitenoberfläche der äußeren Lage (5) anzeigt,
wobei der Marker (30) von einer Nicht-Hautseitenoberflächenseite der äußeren Lage (5) sichtbar ist, wobei
eine Vielzahl der Marker (30) Folgendes einschließt:
einen ersten Marker (31, 32), der auf einer ersten Seite in einer Längsrichtung der äußeren Lage (5) angeordnet ist, und
einen zweiten Marker (33, 34), der auf einer zweiten Seite in der Längsrichtung der äußeren Lage (5) angeordnet ist.

2. Leichtgewichtige Säuglingswindel nach Anspruch 1, wobei
das Paar von Gürtelelementen (20) mit der Nicht-Hautseitenoberfläche der äußeren Lage (5) derart verschlossen ist, dass die Längsrichtung der Gürtelelemente (20) mit einer Querrichtung der äußeren Lage (5) ausgerichtet ist,
das Paar von Gürtelelementen (20) eine Gesamtheit des ersten Markers (31, 32) bedecken und
äußere Bereiche der Gürtelelemente (20) in der Querrichtung der äußeren Lage (5) nach innen gefaltet sind und sich zwischen Bereichen des Saugkörpers (2) in einem Zustand befinden, wo der Saugkörper in der Längsrichtung der äußeren Lage (5) gefaltet wurde.

3. Leichtgewichtige Säuglingswindel nach Anspruch 1 oder 2, wobei
in einem Zustand, wo die äußere Lage (5) in der Längsrichtung der äußeren Lage (5) gefaltet wurde,
zwei Enden der äußeren Lage (5) nicht miteinander ausgerichtet sind, und
der erste Marker (31, 32) und der zweite Marker (33, 34) einen Überlappungsbereich aufweisen, der in einer Richtung überlappt ist, in der Bereiche der gefalteten äußeren Lage (5) überlagert sind, wenn die äußere Lage (5) von der Nicht-Hautseite betrachtet wird.

4. Leichtgewichtige Säuglingswindel nach einem der Ansprüche 1 bis 3, wobei
eine Vielzahl der Marker (30) hinsichtlich Form und Anordnungsposition in Bezug auf eine Mittellinie (CL1), die die äußere Lage (5) in einer Querrichtung der äußeren Lage (5) halbiert, symmetrisch ist.

5. Leichtgewichtige Säuglingswindel nach Anspruch 4, wobei
eine Vielzahl der Marker (30) Folgendes einschließt:
einen Marker auf der einen Seite (31), der eine Verschlussposition für ein Gürtelelement von dem Paar von Gürtelelementen (20) anzeigt, und
einen Marker auf der anderen Seite (32), der eine Verschlussposition für ein anderes Gürtelelement von dem Paar von Gürtelelementen (20) anzeigt, und
der Marker auf der einen Seite und der Marker auf der anderen Seite mit einem Abstand in der Querrichtung der äußeren Lage (5) angeordnet sind.

6. Leichtgewichtige Säuglingswindel nach Anspruch 5, wobei
das Paar von Gürtelelementen (20) mit der Nicht-Hautseitenoberfläche der äußeren Lage (5) derart verschlossen ist, dass die Längsrichtung der Gürtelelemente (20) mit einer Querrichtung der äußeren Lage (5) ausgerichtet ist, und
sich die Größe eines Bereichs (305A) des Markers auf der einen Seite (31), der von dem einen Gürtelelement freigelegt ist, von der Größe eines Bereichs (305B) des Markers auf der anderen Seite (32), der von dem anderen Gürtelelement freigelegt ist, unterscheidet.

7. Leichtgewichtige Säuglingswindel nach Anspruch 5 oder 6, wobei
bezüglich eines inneren Endbereichs (20b), der von jedem der Gürtelelemente (20) vorliegt und der sich innen in der Querrichtung der äußeren Lage (5) befindet,
die leichtgewichtige Säuglingswindel eine Position (p2) aufweist, wo sich der innere Endbereich (20b) zu befinden hat, wenn das Gürtelelement mit der äußeren Lage (5) verschlossen wurde,
bezüglich eines inneren Endbereichs (30b), der von dem Marker auf der einen Seite (31) vorliegt und der sich innen in der Querrichtung der äußeren Lage (5) befindet,
der innere Endbereich (30b) des Markers auf der einen Seite an der Position angeordnet ist, wo sich der innere Endbereich (20b) des einen Gürtelelements zu befinden hat, und
bezüglich eines inneren Endbereichs (30b), der von dem Marker auf der anderen Seite (32) vorliegt und der sich innen in der Querrichtung der äußeren Lage (5) befindet,
der innere Endbereich (30b) des Markers auf der anderen Seite an der Position angeordnet ist, wo sich der innere Endbereich (20b) des anderen Gürtelelements zu befinden hat.

8. Leichtgewichtige Säuglingswindel nach Anspruch 7, wobei
das Paar von Gürtelelementen (20) mit der Nicht-Hautseitenoberfläche der äußeren Lage (5) derart verschlossen ist, dass die Längsrichtung der Gürtelelemente (20) mit einer Querrichtung der äußeren Lage (5) ausgerichtet ist,
der innere Endbereich (30b) des Markers auf der einen Seite (31) einen Überlappungsbereich aufweist, der in der Querrichtung der äußeren Lage (5) mit dem inneren Endbereich (20b) des einen Gürtelelements überlappt ist, und
der innere Endbereich (30b) des Markers auf der anderen Seite (32) einen Überlappungsbereich aufweist, der in der Querrichtung der äußeren Lage (5) mit dem inneren Endbereich (20b) des anderen Gürtelelements überlappt ist.

9. Leichtgewichtige Säuglingswindel nach einem der Ansprüche 5 bis 8, wobei
der Marker auf der einen Seite (31) und der Marker auf der anderen Seite (32) außen in der Querrichtung der äußeren Lage (5) in Bezug auf den Saugkörper angeordnet sind.

10. Leichtgewichtige Säuglingswindel nach einem der Ansprüche 1 bis 9, wobei
der Marker (30) als eine gerade Linie geformt ist, die in einer Querrichtung der äußeren Lage (5) gestreckt ist.

11. Leichtgewichtige Säuglingswindel nach Anspruch 10, wobei
bezüglich eines äußeren Endes (20a), das von jedem der Gürtelelemente (20) vorliegt und das sich außen in einer Längsrichtung der äußeren Lage (5) befindet,
die leichtgewichtige Säuglingswindel eine Position (p1) aufweist, wo sich das äußere Ende zu befinden hat, wenn das Gürtelelement mit der äußeren Lage (5) verschlossen wurde, und
der Marker (30) an der Position angeordnet ist, wo sich das äußere Ende (20a) des Gürtelelements zu befinden hat.

12. Leichtgewichtige Säuglingswindel nach Anspruch 11, wobei
das Paar von Gürtelelementen (20) mit der Nicht-Hautseitenoberfläche der äußeren Lage (5) derart verschlossen ist, dass die Längsrichtung der Gürtelelemente (20) mit einer Querrichtung der äußeren Lage (5) ausgerichtet ist,
sich das äußere Ende (20a) des Gürtelelements näher zu dem Marker (30) in der Längsrichtung der äußeren Lage (5) befindet als das innere Ende (20b) des Gürtelelements ist, und
der Marker (30) einen Bereich aufweist, der sich entlang des äußeren Endes (20a) des Gürtelelements erstreckt.

13. Leichtgewichtige Säuglingswindel nach Anspruch 11 oder 12, wobei
ein Designzeichen (40) auf einer mittleren Seite in der Längsrichtung der äußeren Lage (5) in Bezug auf den Marker (30) bereitgestellt ist und
das Designzeichen (40) von der Nicht-Hautseitenoberflächenseite der äußeren Lage (5) sichtbar ist.

14. Leichtgewichtige Säuglingswindel nach einem der Ansprüche 1 bis 13, wobei
der Marker (30) einen Überlappungsbereich aufweist, der in einer Längsrichtung der äußeren Lage (5) mit einem Endbereich überlappt ist, der von dem Saugkörper (2) vorliegt und sich in der Längsrichtung der äußeren Lage (5) befindet.

15. Leichtgewichtige Säuglingswindel nach einem der Ansprüche 1 bis 14, wobei
der Marker (30) einen Bereich aufweist, der eine Vielzahl von Verschlusspositionen für eines der Gürtelelemente (20) anzeigt.

## Revendications

1. Couche pour nourrissons de faible poids (1) comportant :
un corps absorbant (2) ;
une feuille extérieure (5) agencée sur un côté non orienté vers la peau par rapport au corps absorbant (2) ;
une paire d'éléments formant ceinture (20),
la paire d'éléments formant ceinture (20) comprenant chacun :
un élément de verrouillage (22) sur un côté dans une direction allant dans le sens longitudinal de l'élément formant ceinture et
un élément de verrouillage (22) sur un autre côté dans la direction allant dans le sens longitudinal de l'élément formant ceinture,
Les éléments de la paire d'éléments formant ceinture (20) étant en mesure d'être verrouillés sur une surface du côté non orienté vers la peau de la feuille extérieure (5) au moyen des éléments de verrouillage (22), les éléments de verrouillage étant directement verrouillables sur la feuille extérieure ; et
un marqueur (30) qui indique une position de verrou pour un ou plusieurs parmi les éléments formant ceinture (20) sur la surface du côté non orienté vers la peau de la feuille extérieure (5),
le marqueur (30) étant visible depuis un côté de surface du côté non orienté vers la peau de la feuille extérieure (5), dans laquelle
une pluralité des marqueurs (30) comprend
un premier marqueur (31, 32) agencé sur un premier côté dans une direction allant dans le sens longitudinal de la feuille extérieure (5) et
un deuxième marqueur (33, 34) agencé sur un deuxième côté dans la direction allant dans le sens longitudinal de la feuille extérieure (5).

2. Couche pour nourrissons de faible poids selon la revendication 1, dans laquelle
les éléments de la paire d'éléments formant ceinture (20) sont verrouillés sur la surface du côté non orienté vers la peau de la feuille extérieure (5) de telle sorte que la direction allant dans le sens longitudinal des éléments formant ceinture (20) est alignée sur une direction allant dans le sens transversal de la feuille extérieure (5),
les éléments de la paire d'éléments formant ceinture (20) recouvrent une totalité du premier marqueur (31, 32), et
des parties extérieures des éléments formant ceinture (20) dans la direction allant dans le sens transversal de la feuille extérieure (5) sont pliées vers l'intérieur, et sont situées entre des parties du corps absorbant (2) dans un état dans lequel le corps absorbant a été plié dans la direction allant dans le sens longitudinal de la feuille extérieure (5).

3. Couche pour nourrissons de faible poids selon la revendication 1 ou la revendication 2, dans laquelle
dans une état dans lequel la feuille extérieure (5) a été pliée dans la direction allant dans le sens longitudinal de la feuille extérieure (5),
deux extrémités de la feuille extérieure (5) ne sont pas alignées l'une sur l'autre, et
le premier marqueur (31, 32) et le deuxième marqueur (33, 34) ont une partie de chevauchement chevauchée dans une direction dans laquelle des parties de la feuille extérieure pliée (5) sont superposées, quand la feuille extérieure (5) est vue depuis le côté non orienté vers la peau.

4. Couche pour nourrissons de faible poids selon l'une quelconque des revendications 1 à 3, dans laquelle
les marqueurs d'une pluralité de marqueurs (30) sont symétriques en termes de forme et de position d'agencement par rapport à une ligne centrale (CL1) qui divise en deux la feuille extérieure (5) dans une direction allant dans le sens transversal de la feuille extérieure (5).

5. Couche pour nourrissons de faible poids selon la revendication 4, dans laquelle
une pluralité des marqueurs (30) comprend
un marqueur dit d'un côté (31) indiquant une position de verrouillage pour un élément formant ceinture de la paire d'éléments formant ceinture (20) et
un marqueur dit d'un autre côté (32) indiquant une position de verrouillage pour un autre élément formant ceinture de la paire d'éléments formant ceinture (20), et
le marqueur dit d'un côté et le marqueur dit d'un autre côté sont agencés avec un espace dans la direction allant dans le sens transversal de la feuille extérieure (5).

6. Couche pour nourrissons de faible poids selon la revendication 5, dans laquelle
les éléments de la paire d'éléments formant ceinture (20) sont verrouillés sur la surface du côté non orienté vers la peau de la feuille extérieure (5) de telle sorte que la direction allant dans le sens longitudinal des éléments formant ceinture (20) est alignée sur une direction allant dans le sens transversal de la feuille extérieure (5), et
la taille d'une partie (305A) du marqueur dit d'un côté (31) qui est exposée depuis ledit un élément formant ceinture est différente de la taille d'une partie (305B) du marqueur dit d'un autre côté (32) qui est exposée depuis ledit autre élément formant ceinture.

7. Couche pour nourrissons de faible poids selon la revendication 5 ou la revendication 6, dans laquelle
en ce qui concerne une partie d'extrémité intérieure (20b) qui est de chacun des éléments formant ceinture (20) et qui est situé vers l'intérieur dans la direction allant dans le sens transversal de la feuille extérieure (5),
la couche pour nourrissons de faible poids a une position (p2) où la partie d'extrémité intérieure (20b) doit être placée quand l'élément formant ceinture a été verrouillé sur la feuille extérieure (5),
en ce qui concerne une partie d'extrémité intérieure (30b) qui est du marqueur dit d'un côté (31) et qui est située vers l'intérieur dans la direction allant dans le sens transversal de la feuille extérieure (5),
la partie d'extrémité intérieure (30b) du marqueur dit d'un côté est agencée au niveau de la position où la partie d'extrémité intérieure (20b) dudit un élément formant ceinture doit être placée, et
en ce qui concerne une partie d'extrémité intérieure (30b) qui est du marqueur dit d'un autre côté (32) et qui est située vers l'intérieur dans la direction allant dans le sens transversal de la feuille extérieure (5),
la partie d'extrémité intérieure (30b) du marqueur dit d'un autre côté est agencée au niveau de la position où la partie d'extrémité intérieure (20b) de l'autre élément formant ceinture doit être placée.

8. Couche pour nourrissons de faible poids selon la revendication 7, dans laquelle
les éléments de la paire d'éléments formant ceinture (20) sont verrouillés sur la surface du côté non orienté vers la peau de la feuille extérieure (5) de telle sorte que la direction allant dans le sens longitudinal des éléments formant ceinture (20) est alignée sur une direction allant dans le sens transversal de la feuille extérieure (5),
la partie d'extrémité intérieure (30b) du marqueur dit d'un côté (31) a une partie de chevauchement qui est chevauchée dans la direction allant dans le sens transversal de la feuille extérieure (5) par la partie d'extrémité intérieure (20b) dudit un élément formant ceinture, et
la partie d'extrémité intérieure (30b) du marqueur dit d'un autre côté (32) a une partie de chevauchement qui est chevauchée dans la direction allant dans le sens transversal de la feuille extérieure (5) par la partie d'extrémité intérieure (20b) de l'autre élément formant ceinture.

9. Couche pour nourrissons de faible poids selon l'une quelconque des revendications 5 à 8, dans laquelle
le marqueur dit d'un côté (31) et le marqueur dit d'un autre côté (32) sont agencés vers l'extérieur dans la direction allant dans le sens transversal de la feuille extérieure (5) par rapport au corps absorbant.

10. Couche pour nourrissons de faible poids selon l'une quelconque des revendications 1 à 9, dans laquelle
le marqueur (30) a la forme d'une ligne droite qui est allongée dans une direction allant dans le sens transversal de la feuille extérieure (5).

11. Couche pour nourrissons de faible poids selon la revendication 10, dans laquelle
en ce qui concerne une extrémité extérieure (20a) qui est de chacun des éléments formant ceinture (20) et qui est située vers l'extérieur dans une direction allant dans le sens longitudinal de la feuille extérieure (5),
la couche pour nourrissons de faible poids a une position (p1) où l'extrémité extérieure doit être placée quand l'élément formant ceinture a été verrouillé sur la feuille extérieure (5), et
le marqueur (30) est agencé au niveau de la position où l'extrémité extérieure (20a) de l'élément formant ceinture doit être placée.

12. Couche pour nourrissons de faible poids selon la revendication 11, dans laquelle
les éléments de la paire d'éléments formant ceinture (20) sont verrouillés sur la surface du côté non orienté vers la peau de la feuille extérieure (5) de telle sorte que la direction allant dans le sens longitudinal des éléments formant ceinture (20) est alignée sur une direction allant dans le sens transversal de la feuille extérieure (5),
l'extrémité extérieure (20a) de l'élément formant ceinture est située plus près du marqueur (30) dans la direction allant dans le sens longitudinal de la feuille extérieure (5) que l'extrémité intérieure (20b) de l'élément formant ceinture ne l'est, et
le marqueur (30) a une partie qui s'étend le long de l'extrémité extérieure (20a) de l'élément formant ceinture.

13. Couche pour nourrissons de faible poids selon la revendication 11 ou la revendication 12, dans laquelle
une marque de conception (40) est fournie sur un côté central dans la direction allant dans le sens longitudinal de la feuille extérieure (5) par rapport au marqueur (30) et
la marque de conception (40) est visible depuis le côté de surface du côté non orienté vers la peau de la feuille extérieure (5) .

14. Couche pour nourrissons de faible poids selon l'une quelconque des revendications 1 à 13, dans laquelle
le marqueur (30) a une partie de chevauchement qui est chevauchée dans une direction allant dans le sens longitudinal de la feuille extérieure (5) avec une partie d'extrémité qui est du corps absorbant (2) et qui est située dans la direction allant dans le sens longitudinal de la feuille extérieure (5).

15. Couche pour nourrissons de faible poids selon l'une quelconque des revendications 1 à 14, dans laquelle
le marqueur (30) a une partie qui indique une pluralité de positions de verrouillage pour l'un des éléments formant ceinture (20).
